(19) Europäisches Patentamt
European Patent Office
Office européen des brevets

(11) **EP 3 819 389 B1**

(12) **EUROPEAN PATENT SPECIFICATION**

(45) Date of publication and mention
of the grant of the patent:
**20.03.2024 Bulletin 2024/12**

(21) Application number: **20200749.8**

(22) Date of filing: **05.02.2016**

(51) International Patent Classification (IPC):
*C12Q 1/6886* (2018.01)   *C07H 21/02* (2006.01)
*C07H 21/04* (2006.01)   *C12Q 1/6809* (2018.01)

(52) Cooperative Patent Classification (CPC):
(C-Sets available)
**C12Q 1/6886; C12Q 1/6809;** C12Q 2600/118;
C12Q 2600/156                                (Cont.)

(54) **METHOD OF DETERMINING MICROSATELLITE INSTABILITY**

VERFAHREN ZUR BESTIMMUNG VON MIKROSATELLITENINSTABILITÄT

MÉTHODE DE DÉTERMINATION DE L'INSTABILITÉ MICROSATELLITAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.02.2015 US 201562113340 P
09.02.2015 US 201562113990 P**

(43) Date of publication of application:
**12.05.2021 Bulletin 2021/19**

(62) Document number(s) of the earlier application(s) in
accordance with Art. 76 EPC:
**16747343.8 / 3 253 891**

(73) Proprietor: **Quest Diagnostics Investments LLC
Madison, New Jersey 07940 (US)**

(72) Inventors:
• **Arvai, Kevin J.
San Juan Capistrano, California 92675 (US)**
• **Wang, Yongbao
San Juan Capistrano, California 92675 (US)**
• **Jones, Daniel
San Juan Capistrano, California 92675 (US)**

(74) Representative: **Hoffmann Eitle
Patent- und Rechtsanwälte PartmbB
Arabellastraße 30
81925 München (DE)**

(56) References cited:
• **S. J. SALIPANTE ET AL: "Microsatellite Instability Detection by Next Generation Sequencing", CLINICAL CHEMISTRY, vol. 60, no. 9, 1 September 2014 (2014-09-01), pages 1192-1199, XP055571285, ISSN: 0009-9147, DOI: 10.1373/clinchem.2014.223677**
• **BERND TIMMERMANN ET AL: "Somatic Mutation Profiles of MSI and MSS Colorectal Cancer Identified by Whole Exome Next Generation Sequencing and Bioinformatics Analysis", PLOS ONE, vol. 5, no. 12, 22 December 2010 (2010-12-22), page e15661, XP055385156, DOI: 10.1371/journal.pone.0015661**
• **T.-M. KIM ET AL: "A Genome-wide View of Microsatellite Instability: Old Stories of Cancer Mutations Revisited with New Sequencing Technologies", CANCER RESEARCH, vol. 74, no. 22, 4 November 2014 (2014-11-04), pages 6377-6382, XP055738231, US ISSN: 0008-5472, DOI: 10.1158/0008-5472.CAN-14-1225**
• **CIOMBOR KRISTEN K. ET AL: "How Can Next-Generation Sequencing (Genomics) Help Us in Treating Colorectal Cancer?", CURRENT COLORECTAL CANCER REPORTS, vol. 10, no. 4, 17 August 2014 (2014-08-17), pages 372-379, XP055792422, Boston ISSN: 1556-3790, DOI: 10.1007/s11888-014-0244-3**
• **MONIKA ZAVODNA ET AL: "The Accuracy, Feasibility and Challenges of Sequencing Short Tandem Repeats Using Next-Generation Sequencing Platforms", PLOS ONE, vol. 9, no. 12, 1 December 2014 (2014-12-01), page e113862, XP055482447, DOI: 10.1371/journal.pone.0113862**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 819 389 B1

(52) Cooperative Patent Classification (CPC): (Cont.)

C-Sets
**C12Q 1/6809, C12Q 2535/122**

**Description**

**FIELD OF THE INVENTION**

The present invention relates generally a method of determining microsatellite instability (MSI) of a DNA sample as defined in the appended claims.

**BACKGROUND OF THE INVENTION**

The following discussion of the background of the invention is merely provided to aid the reader in understanding the invention and is not admitted to describe or constitute prior art to the present invention.

Endometrial cancer (EC) encompasses the common endometrioid histologic subtype, with variable clinical outcomes, and the less common papillary serous/clear cell carcinoma (PSC), with uniformly adverse prognosis. EC arises from the lining of the uterus, and it is the fourth most common malignancy among women in the United States. In 2013, there were an estimated 49,500 new cases diagnosed and 8,200 deaths resulting from EC. Most patients present with low-grade, early-stage disease. However, the majority of patients with more aggressive, high-grade tumors who have disease spread beyond the uterus will progress within 1 year.

Endometrial cancers have been broadly classified into two groups. Type I endometrioid tumors are linked to oestrogen excess, obesity, and hormone-receptor positivity, and individuals with type I tumors generally have favorable prognosis compared with type II. Type II represents primarily serous tumors that are more common in older, non-obese women and have a comparatively worse outcome. Early-stage endometrioid cancers are often treated with adjuvant radiotherapy, whereas serous tumors are treated with chemotherapy. Therefore, proper subtype classification is crucial for selecting appropriate adjuvant therapy, but diagnostic solutions for addressing this need are limited.

The primary unmet diagnostic need in EC is the identification of cases with endometrioid histology and low-stage that have risk of recurrence and would benefit from adjuvant chemotherapy.

A multicenter Uterine Corpus Endometrial Carcinoma (UCEC) study recently employed expression and genomic microarrays, methylation profiling, and next-generation sequencing (NGS) to address this need. Based primarily on the sequencing and microarray data, the study identified four distinct molecular clusters of EC. (The Cancer Genome Atlas Research Network, Integrated genomic characterization of endometrial carcinoma, 00 NATURE, 1-8 (2013)). These clusters included 1) a group of POLE-mutated cases associated with an extremely high mutation rate and favorable prognosis and 2) a group of mostly, but not exclusively, PSC cases associated with TP53 mutations, frequent genomic copy-number (CN) changes, and poor prognosis. However, most of the cases (155/232, 66.8%) were 3) endometrioid EC with unmutated TP53 and few CN changes, or 4) cases exhibiting microsatellite instability (MSI). These last 2 groups had more variable outcomes. Salipante, Stephen J., et al. describe that the fraction of unstable microsatellite markers calculated from sequencing data correlated with the number of unstable loci detected by conventional MSI-PCR resting and conclude that NGS data can enable highly accurate detection of MSI, even from limited capture designs. (Salipante, Stephen J., et al. "Microsatellite instability detection by next generation sequencing." Clinical chemistry 60.9 (2014): 1192-1199.

But in spite of such comprehensive studies, the defined molecular clusters or subgroup specific mutations are not used in determining prognosis. The compositions and methods disclosed herein were elucidated by examination of how mutation pattern, MSI status, total number of CN alterations, and mutation load interact. The present disclosure provides a cluster prediction model that was developed based on a large clinical data set and additional findings related to new prognostic indicators of recurrence in low-stage endometrioid tumors. Moreover, the disclosed model and methods can be applied to other disease states in order to identify and validate molecular markers of prognosis.

**SUMMARY OF THE INVENTION**

The invention is defined in the following items which correspond to the appended claims:

1. A method of determining microsatellite instability (MSI) of a DNA sample comprising,

   a) obtaining a tumor sample and a normal sample from a subject, wherein each sample comprises DNA;

   b) sequencing a microsatellite location in the DNA of each sample using next generation sequencing (NGS);

   c) identifying tandem repeats in the sequences;

   d) extracting coverage and total read values from the sequences comprising tandem repeats;

e) calculating the divergence of the normal sample and the tumor sample;

f) calculating the delta divergence for each nucleotide position;

g) calculating the sum of all delta divergence values; wherein the value obtained from the sum of all delta divergence values represents the quantification of sequence divergence between the normal sample and the tumor sample at the sequenced microsatellite location..

2. The method of item 1, wherein more than one microsatellite location is sequenced at a time.

3. The method of and of items 1 or 2 further comprising testing the sample for a mutation in the ESR1 gene.

4. The method of item 3, wherein the mutation in the ESR1 gene is a Y537 substitution.

5. The method of item 3, wherein the mutation in the ESR1 gene is an in-frame deletion.

6. The method of item 3, wherein the mutation in the ESR1 gene results in an amino acid substitution in the ESR1 ligand binding domain (LBD), optionally, wherein the mutation in the ESR1 gene results in an in-frame deletion in the ESR1 LBD.

7. The method of item 3, wherein the mutation in the ESR1 gene is GKC415del.

8. The method of any one of items 1-7 further comprising testing the sample for a mutation in the CDSE1 gene and the SGK1 gene.

9. The method of any one of items 1-8, wherein the subject has a tumor or cancer.

10. The method of item 9, wherein the cancer is endometrial cancer.

11. The method of item 10, wherein the endometrial cancer is stage I/II endometrial cancer.

12. The method of any one of items 10 or 11, wherein the subject has previously undergone surgery to remove the tumor or cancer.

13. The method of any one of items 10-12, wherein the subject is non-symptomatic or in remission.

The present description provides methods and compositions for determining prognosis of individuals with endometrial cancer (EC) and other similar conditions. In particular, the description provides methods of prognosing survival, predicting recurrence, and guiding treatment based on ESR1 mutation status in a subgroup of subjects with EC. The invention may be used alone, or in combination with other clinical symptoms, diagnostics, or indicators, for determining the prognosis of an individual with EC or other similar conditions.

Additionally, the present description provides methods of developing prognostic models in multiple disease states based on molecular clustering data.

Accordingly, in one aspect, the present description provides a method of determining the risk of recurrence of endometrial cancer in a subject comprising: obtaining a sample from the subject; testing the sample for a mutation in the ESR1 gene; and indicating the subject is not at risk of recurrence if the ESR1 gene is not mutated or the subject is at risk of recurrence if the ESR1 gene is mutated.

In some embodiments, the endometrial cancer is stage VII, and in some embodiments, the stage I/II endometrial cancer may further comprise low copy number changes and microsatellite instability.

In some embodiments, the mutation in the ESR1 gene is a Y537 substitution, and in other embodiments, the mutation in the ESR1 gene is an in-frame deletion.

In some embodiments, the method of determining the risk of recurrence of endometrial cancer in a subject further comprises administering to the subject a compound for treating endometrial cancer when the ESR1 gene is mutated. In some embodiments, the subject may have previously undergone surgery to remove an endometrial tumor, and in some embodiments, the subject may be non-symptomatic or the subject may be in remission.

In another aspect, the present description provides a method of predicting recurrence in a subject with endometrial cancer comprising: obtaining a sample from the subject; testing the sample for a mutation in the ESR1 gene; and indicating the subject will experience cancer recurrence if the ESR1 gene is mutated and the subject will not experience cancer recurrence if the ESR1 gene is not mutated.

In some embodiments, the endometrial cancer is stage VII, and in some embodiments, the stage I/II endometrial cancer may further comprise low copy number changes and microsatellite instability.

In some embodiments, the mutation in the ESR1 gene is a Y537 substitution, and in other embodiments, the mutation in the ESR1 gene is an in-frame deletion.

In some embodiments, the method of predicting recurrence in a subject with endometrial cancer may further comprise administering to the subject a compound for treating endometrial cancer when the ESR1 gene is mutated. In some embodiments, the subject may have previously undergone surgery to remove an endometrial tumor, and in some embodiments, the subject may be non-symptomatic or the subject may be in remission.

In another aspect, the present description provides a method for guiding treatment in a subject with endometrial cancer comprising: obtaining a sample from the subject; testing the sample for a mutation in the ESR1 gene; and indicating the subject should receive chemotherapy if the ESR1 gene is mutated.

In some embodiments, the endometrial cancer is stage VII, and in some embodiments, the stage I/II endometrial cancer may further comprise low copy number changes and microsatellite instability.

In some embodiments, the mutation in the ESR1 gene is a Y537 substitution, and in other embodiments, the mutation in the ESR1 gene is an in-frame deletion.

In some embodiments, the method of guiding treatment in a subject with endometrial cancer may involve a subject that has previously undergone surgery to remove an endometrial tumor, and in some embodiments, the subject may be non-symptomatic or the subject may be in remission.

In one aspect, the present description provides a method of determining the risk of recurrence of endometrial cancer in a subject comprising: obtaining a sample from the subject; testing the sample for a mutation in the CSDE1 gene; and indicating the subject is not at risk of recurrence if the CSDE1 gene is not mutated or the subject is at risk of recurrence if the CSDE1 gene is mutated.

In some embodiments, the endometrial cancer is stage VII, and in some embodiments, the stage I/II endometrial cancer may further comprise low copy number changes and microsatellite instability.

In some embodiments, the method of determining the risk of recurrence of endometrial cancer in a subject further comprises administering to the subject a compound for treating endometrial cancer when the CSDE1 gene is mutated. In some embodiments, the subject may have previously undergone surgery to remove an endometrial tumor, and in some embodiments, the subject may be non-symptomatic or the subject may be in remission.

In another aspect, the present description provides a method of predicting recurrence in a subject with endometrial cancer comprising: obtaining a sample from the subject; testing the sample for a mutation in the CSDE1 gene; and indicating the subject will experience cancer recurrence if the CSDE1 gene is mutated and the subject will not experience cancer recurrence if the CSDE1 gene is not mutated.

In some embodiments, the endometrial cancer is stage VII, and in some embodiments, the stage I/II endometrial cancer may further comprise low copy number changes and microsatellite instability.

In some embodiments, the method of predicting recurrence in a subject with endometrial cancer may further comprise administering to the subject a compound for treating endometrial cancer when the CSDE1 gene is mutated. In some embodiments, the subject may have previously undergone surgery to remove an endometrial tumor, and in some embodiments, the subject may be non-symptomatic or the subject may be in remission.

In another aspect, the present description provides a method for guiding treatment in a subject with endometrial cancer comprising: obtaining a sample from the subject; testing the sample for a mutation in the CSDE1 gene; and indicating the subject should receive chemotherapy if the CSDE1 gene is mutated.

In some embodiments, the endometrial cancer is stage I/II, and in some embodiments, the stage I/II endometrial cancer may further comprise low copy number changes and microsatellite instability.

In some embodiments, the method of guiding treatment in a subject with endometrial cancer may involve a subject that has previously undergone surgery to remove an endometrial tumor, and in some embodiments, the subject may be non-symptomatic or the subject may be in remission.

in one aspect, the present description provides a method of determining the risk of recurrence of endometrial cancer in a subject comprising: obtaining a sample from the subject; testing the sample for a mutation in the SGK1 gene; and indicating the subject is not at risk of recurrence if the SGK1 gene is not mutated or the subject is at risk of recurrence if the SGK1 gene is mutated.

In some embodiments, the endometrial cancer is stage I/II, and in some embodiments, the stage I/II endometrial cancer may further comprise low copy number changes and microsatellite instability.

In some embodiments, the method of determining the risk of recurrence of endometrial cancer in a subject further comprises administering to the subject a compound for treating endometrial cancer when the SGK1 gene is mutated. In some embodiments, the subject may have previously undergone surgery to remove an endometrial tumor, and in some embodiments, the subject may be non-symptomatic or the subject may be in remission.

In another aspect, the present description provides a method of predicting recurrence in a subject with endometrial cancer comprising: obtaining a sample from the subject; testing the sample for a mutation in the SGK1 gene; and indicating the subject will experience cancer recurrence if the SGK1 gene is mutated and the subject will not experience cancer recurrence if the SGK1 gene is not mutated.

In some embodiments, the endometrial cancer is stage I/II, and in some embodiments, the stage I/II endometrial cancer may further comprise low copy number changes and microsatellite instability.

In some embodiments, the method of predicting recurrence in a subject with endometrial cancer may further comprise administering to the subject a compound for treating endometrial cancer when the SGK1 gene is mutated. In some embodiments, the subject may have previously undergone surgery to remove an endometrial tumor, and in some embodiments, the subject may be non-symptomatic or the subject may be in remission.

In another aspect, the present description provides a method for guiding treatment in a subject with endometrial cancer comprising: obtaining a sample from the subject; testing the sample for a mutation in the SGK1 gene; and indicating the subject should receive chemotherapy if the SGK1 gene is mutated.

In some embodiments, the endometrial cancer is stage I/II, and in some embodiments, the stage I/II endometrial cancer may further comprise low copy number changes and microsatellite instability.

In some embodiments, the method of guiding treatment in a subject with endometrial cancer may involve a subject that has previously undergone surgery to remove an endometrial tumor, and in some embodiments, the subject may be non-symptomatic or the subject may be in remission.

In one aspect, the present invention provides a method of detecting microsatellite instability using next generation sequencing, comprising: obtaining a tumor sample and a normal sample; sequencing a microsatellite location of the tumor sample and the normal sample using next generation sequencing; identifying tandem repeats in the sequences; extracting coverage and total read values from the sequences comprising tandem repeats; calculating the divergence of the normal sample and the tumor sample; calculating the delta divergence for each nucleotide position; calculating the sum of all delta divergence values; wherein the value obtained from the sum of all delta divergence values represents the quantification of sequence divergence between the normal sample and the tumor sample at the sequenced microsatellite location. In some embodiments, more than one microsatellite location is sequenced at a time. In some embodiments, the method of detecting microsatellite instability may be carried out on historical datasets rather than individual samples. In some embodiment, the method of detecting microsatellite instability may be carried out in order to identify previously unknown microsatellite loci.

In one aspect, the present description provides a method of determining cancer subtypes, comprising: a dataset comprising known mutations and genetic alterations in a specific cancer; converting the known mutations and alterations into quantifiable features according to a Naïve-Bayes model; selecting the most predictive features according to the feature's chi square value; identifying a cancer subtype according to the selected predictive features.

In some embodiments, the known mutations and genetic alterations comprise gene mutations and microsatellite status.

In some embodiments, the identified subtype may indicate an increased risk of recurrence, a decreased likelihood of progression-free survival. Additionally, identifying the subtype of cancer may be used to guide treatment of a subject with the identified subtype of cancer. In some embodiments, the subject is administered chemotherapy following surgery to remove a primary tumor according to the identification of a specific subtype.

## BRIEF DESCRIPTION OF FIGURES

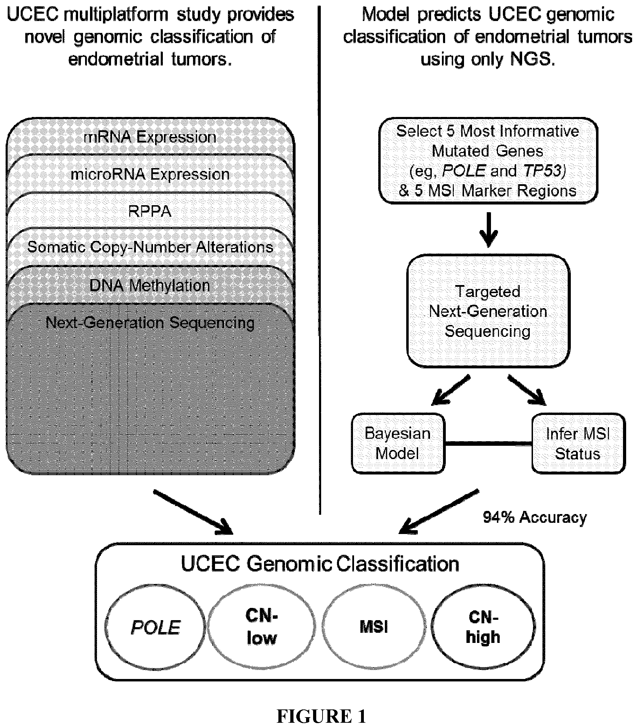

**Figure 1** shows genomic classifications for endometrial tumors base on multiplatform testing and clustering algorithms (left), and a model (right) of predicting the same genomic classifications with 94% accuracy using binary mutation status of five mutated genes and microsatellite instability status.

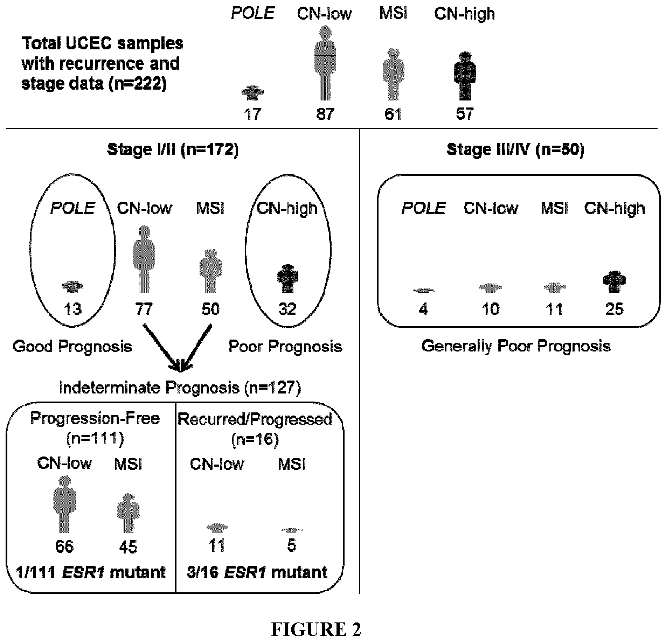

**Figure 2** shows a visual representation highlighting the distribution of genomic classifications in endometrial cancer. The majority of stage I/II cases (74%) were classified as either CN-low or MSI. The genes mutated at a higher rate in recurred cases (e.g. ESR1) are found in this indeterminate subset. The stage III/IV cases are dominated by CN-high classification and have a generally poor prognosis.

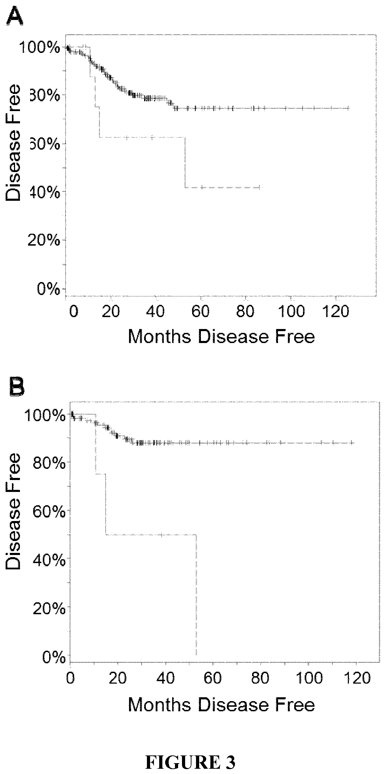

**Figure 3** shows Kaplan-Meier curves showing disease-free survival of endometrial cancer. Panel A shows all cases while Panel B only depicts a subset consisting of stage I/II cases. The lighter, lower line depicts cases with mutant ESR1 and the darker, higher line depicts cases with wild-type ESR1.

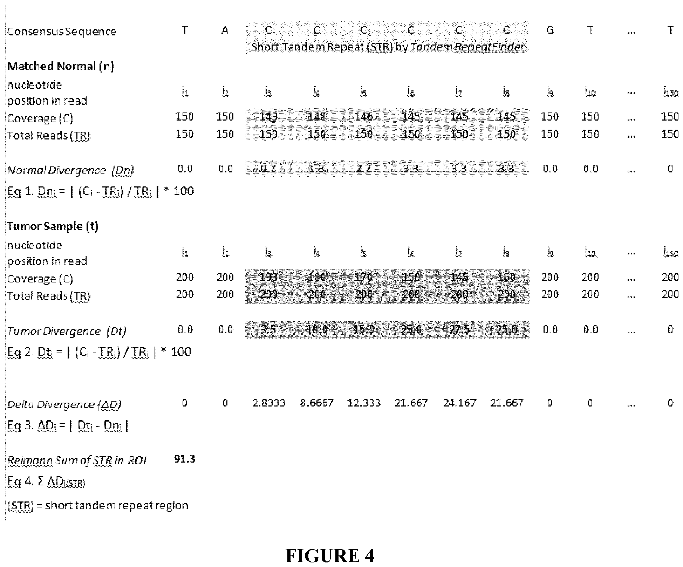

**Figure 4** illustrates exemplary methods for quantifying microsatellite instability from NGS reads.

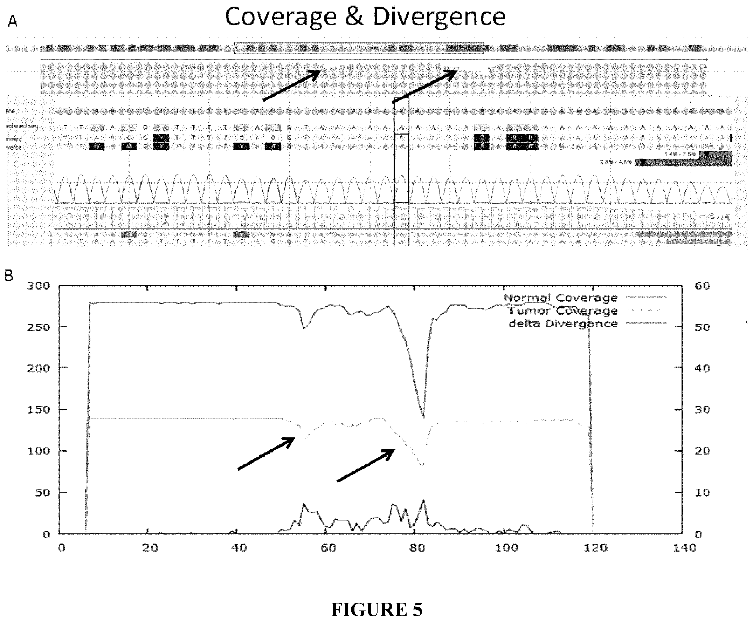

**Figure 5** shows a histogram image from JSI-SeqNext software (A) showing sequencing coverage of a normal sample, the black arrows indicate two microsatellite areas. Also shown is an image produced from the MSI detection method (B). The nucleotide position (i) of the amplicon/region of interest is located on the x axis. Black arrows serve as a reference for accurate representation of the coverage histogram. Normal sample coverage (red, upper line) and tumor sample coverage (green, middle line) are measured in number of nucleotides (y1 axis). Delta divergence (blue, lower line) (Eq. 3) is indicated by the integer values on the y2 axis.

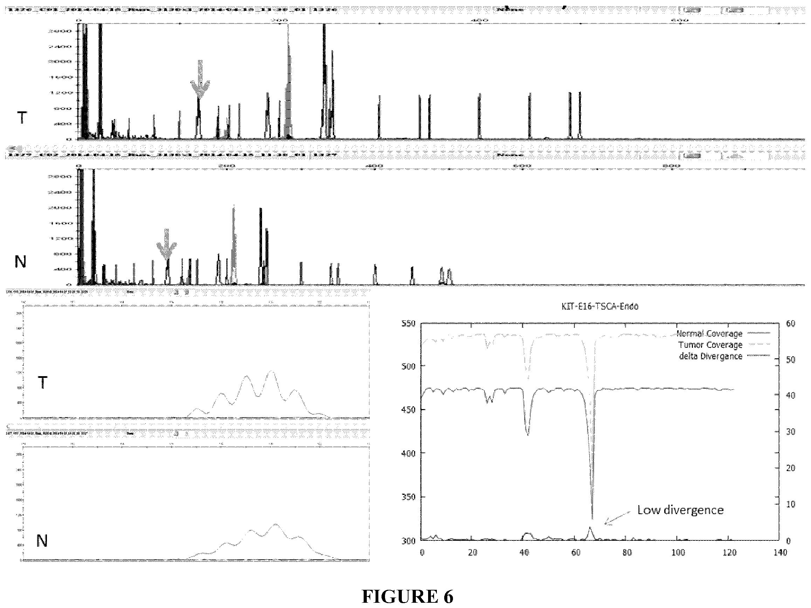

**Figure 6** shows an example of a sample showing a microsatellite stable region of interest (ROI). The tumor (T) and normal (N) samples were run by capillary electrophoresis for five MSI markers (top). A unimodal peak is visible at high power (bottom left) in both the tumor and normal samples, indicating a stable microsatellite (MSS) pattern. The MSI detection method (bottom right) detects a low divergence (blue, lower line), confirming the results.

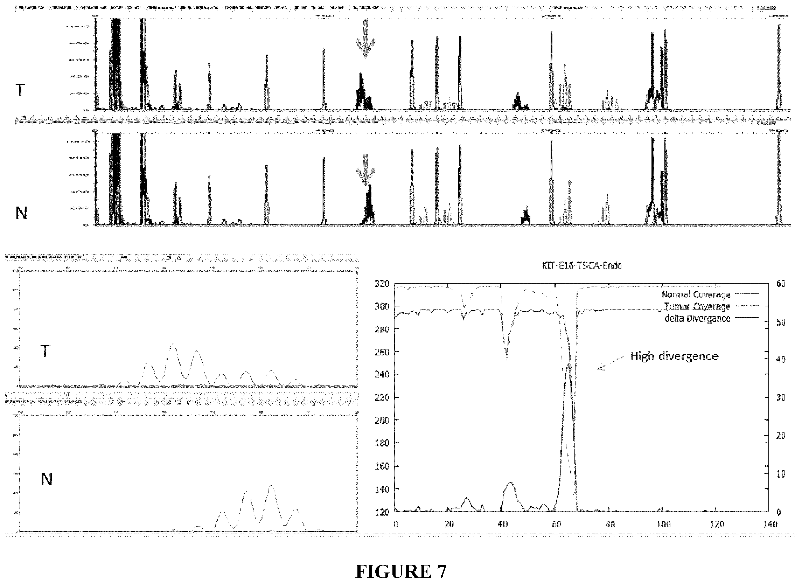

**Figure 7** shows an example of a sample showing a microsatellite instable region of interest (ROI). The tumor (T) and normal (N) samples were run by capillary electrophoresis for five MSI markers (top). A slight bimodal peak is visible at high power (bottom left) in both the tumor and normal samples, indicating a MSI in this region. The MSI detection method (bottom right) detects a high divergence (blue, lower line), confirming the results.

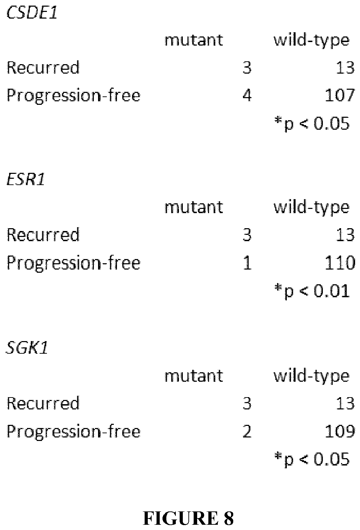

**Figure 8** shows results of the Fisher's exact test for each of the three genes where mutation was significantly correlate with recurrence in the Stage I/II, CN-low/MSI subgroup.

## DETAILED DESCRIPTION

The present invention is defined in the appended claims.

Provided herein are methods for detecting ESR1 mutations in stage I/II endometrial cancer and predicting an individual's prognosis according to the mutation status. Additionally, methods for diagnosis, prognosis, management, and treatment decisions in patients with ESR1 mutated-endometrial cancer or similar conditions. More generally, the present description provides methods and models for diagnosing and prognosing cancer patients, and specifically those patients with endometrial cancer. The disclosed methods may be used to improve the prognosis of an individual based on the individual's specific molecular or genetic profile. Determining the disease prognosis or likelihood of recurrence in an individual may improve treatment outcomes and increase survival.

Also provided herein are methods of developing a model to identify molecular clusters that may be used in the diagnosis or prognosis of disease. Developing such a model may comprise incorporating data related to mRNA expression, microRNA expression, somatic copy number alterations, DNA methylation, sequencing (including Sanger and Next-Generation sequencing), and reverse phase protein arrays, among other molecular diagnostic techniques. By utilizing the steps of data analysis as disclosed herein, one of skill in the art can develop models useful for predicting prognosis of various diseases based on molecular cluster profiles.

It is to be understood that methods are not limited to the particular embodiments described, and as such may vary. It is also to be understood that the terminology used herein is for the purpose of describing particular embodiments only, and is not intended to be limiting. The scope of the present technology will be limited only by the appended claims.

As used herein, "about" means plus or minus 10%.

As used herein, "optional" or "optionally" means that the subsequently described event or circumstance may or may not occur, and that the description includes instances where said event or circumstance occurs and instances where it does not.

As used herein, the term "sample," "test sample," or "biological sample" refers to any liquid or solid material derived from an individual believed to have endometrial cancer. In preferred embodiments, a test sample is obtained from a biological source, such as cells in culture or a tissue or fluid sample from an animal, most preferably, a human. Preferred samples of the invention include, but are not limited to, biopsy, aspirates, plasma, serum, whole blood, blood cells, lymphatic fluid, cerebrospinal fluid, synovial fluid, urine, saliva, and skin or other organs (e.g. biopsy material). The term "patient sample" as used herein may also refer to a tissue sample obtained from a human seeking diagnosis or treatment of endometrial cancer or a related condition or disease. Each of these terms may be used interchangeably.

As used herein, "having an increased risk" means a subject that is identified as having a higher than normal chance of developing cancer, compared to the general population. In addition, a subject who has had, or who currently has, cancer is a subject who has an increased risk for developing cancer, as such a subject may continue to develop cancer. Subjects who currently have, or who have had, a tumor also have an increased risk for tumor metastases.

As used herein, "determining a prognosis" refers to the process in which the course or outcome of a condition in a patient is predicted. The term "prognosis" does not refer to the ability to predict the course or outcome of a condition with 100% accuracy. Instead, the term refers to identifying an increased or decreased probability that a certain course or outcome will occur in a patient exhibiting a given condition/marker, when compared to those individuals not exhibiting the condition. The nature of the prognosis is dependent upon the specific disease and the condition/marker being assessed. For example, a prognosis may be expressed as the amount of time a patient can be expected to survive, the likelihood that the disease goes into remission or experience recurrence, or to the amount of time the disease can be expected to remain in remission before recurrence.

Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon.

Disclosed herein are methods for diagnostic and prognostic evaluation of cancer. Also disclosed are methods of treating cancer. In one aspect, the expression of genes (and/or the stability of microsatellites) or proteins are determined in different subjects for which either diagnosis or prognosis information is desired, in order to provide cancer profiles.

Within the cancer tissue, different expression profiles may be indicative of different prognosis states (i.e. good long term survival prospects or poor long term survival prospects, for example). By comparing profiles of cancer tissue in different states, information regarding which genes are important (including both up- and down-regulation and/or mutation of genes) in each of these states is obtained. The identification of sequences that are differentially expressed in cancer tissue, as well as differential expression resulting in different prognostic outcomes is clinically invaluable for determining patient treatment.

A particular treatment regime may be evaluated according to whether it will improve a given patient's outcome, meaning it will reduce the risk of recurrence or increase the likelihood of progression-free survival. For example, early-stage endometrioid cancers are generally treated with adjuvant radiotherapy, whereas serous tumors, which are usually more aggressive, are treated with chemotherapy. However, certain subsets of individuals with early-stage endometrioid tumors will experience disease recurrence and potentially death, even after being treated with adjuvant radiotherapy. Diagnostic and prognostic methods that are capable of identifying susceptible subpopulations are paramount for determining the proper course of treatment for these individuals.

**Genetic Profiles in Cancer:**

In some embodiments, the present disclosure provides a method for determining prognosis in an individual with cancer according to a particular molecular profile. A profile may include, but is not limited to, microsatellite status, copy-number (CN) status, and the mutation status of specific oncogenes, for instance, POLE, PTEN, TP53, FBXW7, RPL22, FGFR2, PIK3CA, and/or ESR1, CSDE1, and/or SGK1. Individuals may be stratified into subgroups or subsets based on the combination of these factors. For instance, in endometrial cancer, a subgroup may be defined as being POLE-mutated and having a high mutation rate. This group may have a favorable prognosis, while another subgroup typified by having TP53 mutations and high CN changes may have a poor prognosis.

*Microsatellite Stability*

A microsatellite locus is a region of genomic DNA with simple tandem repeats that are repetitive units of one to five base pairs in length. Hundreds of thousands of such microsatellite loci are dispersed throughout the human genome. Microsatellite loci are classified based on the length of the smallest repetitive unit. For example, loci with repetitive units of 1 to 5 base pairs in length are termed "mono-nucleotide", "di-nucleotide", "tri-nucleotide", "tetra-nucleotide", and "penta-nucleotide" repeat loci, respectively.

Microsatellite instability (MSI) is a genetic defect whereby localized repetitive stretches of the genome (termed "microsatellites") vary in size due to polymerase slippage during DNA replication. MSI is characteristic of a subset of human cancers, where it has diagnostic, prognostic and therapeutic consequences. MSI-positive colorectal carcinoma (CRC) has favorable prognosis compared to other subtypes and can have inferior response to adjuvant chemotherapy.

Each microsatellite locus of normal genomic DNA for most diploid species, such as genomic DNA from mammalian species, consists of two alleles at each locus. The two alleles can be the same or different from one another in length and can vary from one individual to the next. Microsatellite alleles are normally maintained at constant length in a given individual and its descendants; but, instability in the length of microsatellites has been observed in some tumor types. This form of genomic instability in tumor is termed microsatellite instability.

The molecular basis of MSI is mutation, gene deletion or epigenetic silencing of one or more of the mismatch repair (MMR) proteins, including *MLH1, MSH2, MSH6* and *PMS2*. These MMR genetic alterations can occur as germline mutations, as in Lynch syndrome/hereditary non-polyposis colorectal carcinoma, or as sporadic changes occurring during tumor development.

Universal screening for Lynch syndrome in patients with colon cancer is now recommended; however, this screening may also be appropriate in endometrial cancer. Lynch screening strategies include MSI detection by PCR and/or immunohistochemistry (IHC) to detect loss of expression of the mismatch repair proteins in tissue sections.

Parallel testing with both MSI PCR and IHC offers the most robust yield.

The predominant method for detecting MSI PCR is a comparison of the amplification pattern of 5 microsatellite loci in paired samples of macrodissected normal/non-neoplastic tissues and tumor tissue. The 5 microsatellites used for PCR analysis are mostly commonly the NCI-designated panel, comprising two mononucleotide loci big Adenine Tract, *BAT-25* and *BAT-26,* and three dinucleotide loci (*D2S123, D5S346,* and *D17S250).*

Other methods of detection of MSI are known in the art, and one of skill in the art can determine which method to use with those methods disclosed herein. For instance, DNA sequencing represents an alternate method for detecting MSI by directly assessing the length of microsatellites in normal-tumor paired samples. By categorizing microsatellites by DNA sequencing, a single assay can be designed to simultaneously detect MSI and mutation status. However, a method to translate raw sequence reads into MSI status has not been well-validated. Other methods have been reported for detecting microsatellites in sequencing data that are distinct from the method reported here including Beifant et al, 2013 and Kim, Laird & Park, 2013, which are incorporated herein by reference.

MSI is diagnosed whenever at least 2 of the 5 loci show size shifts in the tumor sample as compared to the non-neoplastic tissue. This is usually assessed by visual interpretation of capillary gel electrophoresis electropherograms. As such, MSI calls by PCR can occasionally be subjective, especially when clear-cut abnormal PCR amplification is seen in only 1 of 5 tested loci, a finding termed MSI-low.

Many informative microsatellite loci have been identified and recommended for MSI testing, and therefore one of skill in the art will be able to determine what combination of microsatellites is appropriate for testing in a specific situation (i.e. those microsatellites associated with a specific type of cancer). Multiple markers can be used to increase the power of detection, and can be used in conjunction with the disclosed methods. To increase the specificity of an MSI assay for any given type of cancer, it has been recommended that the panel of at least five highly informative microsatellite loci. Increased information yielded from amplifying and analyzing greater numbers of loci generally results in increased confidence and accuracy in interpreting test results.

In performing next-generation sequencing (NGS) studies on tumors, it has been determined that tumor cases with MSI produce a pattern of read dropout during the sequence alignment process that is characteristic and distinct from other sequence alterations that could be observed. Disclosed herein are newly designed and validated processes to detect this pattern of alignment that allow accurate determination of MSI status using NGS data.

### *Copy-Number Changes*

Copy-number changes (CNs) are a form of structural variation of the genome that result in a cell having an abnormal number of copies of one or more sections of DNA. DNA regions may be deleted or duplicated on a given chromosome.

CNs are generally stable and heritable, but may also arise de novo during development. Like other types of genetic variation, some CNs have been associated with susceptibility or resistance to disease, and gene copy number is often be elevated in cancer, for instance, endometrial cancer.

### *POLE*

POLE (HGNC: 9177, Gene ID: 5426, NG_033840.1) encodes the catalytic subunit of DNA polymerase epsilon. The enzyme is involved in DNA repair and chromosomal DNA replication. Mutations in this gene have been associated with colorectal cancer, endometrial cancer, facial dysmorphism, immunodeficiency, livedo, and short stature.

### *PTEN*

PTEN (HGNC: 9588, Gene ID: 5728, NG_000305.3) was identified as a tumor suppressor that is mutated in a large number of cancers at high frequency. The protein encoded this gene is a phosphatidylinositol-3,4,5-trisphosphate 3-phosphatase. It contains a tensin like domain as well as a catalytic domain similar to that of the dual specificity protein tyrosine phosphatases. Unlike most of the protein tyrosine phosphatases, this protein preferentially dephosphorylates phosphoinositide substrates. It negatively regulates intracellular levels of phosphatidylinositol-3,4,5-trisphosphate in cells and functions as a tumor suppressor by negatively regulating AKT/PKB signaling pathway.

### *TP53*

TP53 (HGNC: 11998, Gene ID: 7157, NG_017013.2) encodes a tumor suppressor protein containing transcriptional activation, DNA binding, and oligomerization domains. The encoded protein responds to diverse cellular stresses to regulate expression of target genes, thereby inducing cell cycle arrest, apoptosis, senescence, DNA repair, or changes in metabolism. Mutations in this gene are associated with a variety of human cancers, including hereditary cancers such as Li-Fraumeni syndrome. Alternative splicing of this gene and the use of alternate promoters result in multiple transcript variants and isoforms. Additional isoforms have also been shown to result from the use of alternate translation initiation codons

*FGFR2*

FGFR2 (HGNC: 3689, Gene ID: 2263, NG_012449.1) encodes fibroblast growth factor receptor 2, a member of the fibroblast growth factor receptor family with an amino acid sequence that is highly conserved between members and throughout evolution. FGFR family members differ from one another in their ligand affinities and tissue distribution. A full-length representative protein consists of an extracellular region, composed of three immunoglobulin-like domains, a single hydrophobic membrane-spanning segment and a cytoplasmic tyrosine kinase domain. The extracellular portion of the protein interacts with fibroblast growth factors, setting in motion a cascade of downstream signals, ultimately influencing mitogenesis and differentiation. This particular family member is a high-affinity receptor for acidic, basic and/or keratinocyte growth factor, depending on the isoform. Mutations in this gene are associated with Crouzon syndrome, Pfeiffer syndrome, Craniosynostosis, Apert syndrome, Jackson-Weiss syndrome, Beare-Stevenson cutis gyrata syndrome, Saethre-Chotzen syndrome, syndromic craniosynostosis, and endometrial cancer, among other pathological conditions. Multiple alternatively spliced transcript variants encoding different isoforms have been noted for this gene.

*PIK3CA*

The PIK3CA gene (HGNC: 8975, Gene ID: 5290, NG_0121113.2) encodes the p110$\alpha$ protein or phosphatidylinositol-4,5-bisphosphate 3-kinase, catalytic subunit alpha. Recent evidence has shown that the PIK3CA gene is mutated in a range of human cancers including, but not limited to, cervical cancer and endometrial cancer. Phosphatidylinositol 3-kinase is composed of an 85 kDa regulatory subunit and a 110 kDa catalytic subunit. The protein encoded by this gene represents the catalytic subunit, which uses ATP to phosphorylate PtdIns, PtdIns4P and PtdIns(4,5)P2.

*FBXW7*

The FBXW& gene (HGNC: 16712, Gene ID: 55294, NG_029466.1) encodes a member of the F-box protein family which is characterized by an approximately 40 amino acid motif, the F-box. The F-box proteins constitute one of the four subunits of ubiquitin protein ligase complex called SCFs (SKP1-cullin-F-box), which function in phosphorylation-dependent ubiquitination. The F-box proteins are divided into 3 classes: Fbws containing WD-40 domains, Fbls containing leucine-rich repeats, and Fbxs containing either different protein-protein interaction modules or no recognizable motifs. The protein encoded by this gene was previously referred to as FBX30, and belongs to the Fbws class; in addition to an F-box, this protein contains 7 tandem WD40 repeats. This protein binds directly to cyclin E and probably targets cyclin E for ubiquitin-mediated degradation. Mutations in this gene are detected in ovarian and breast cancer cell lines, implicating the gene's potential role in the pathogenesis of human cancers. Multiple transcript variants encoding different isoforms have been found for this gene.

*RPL22*

The RPL22 gene(HGNC: 10315, Gene ID: 6146, NC_000001.11) encodes a cytoplasmic ribosomal protein that is a component of the 60S subunit. The protein belongs to the L22E family of ribosomal proteins. Its initiating methionine residue is post-translationally removed. The protein can bind specifically to Epstein-Barr virus-encoded RNAs (EBERs) 1 and 2. The mouse protein has been shown to be capable of binding to heparin. Transcript variants utilizing alternative polyA signals exist. As is typical for genes encoding ribosomal proteins, there are multiple processed pseudogenes of this gene dispersed through the genome. It was previously thought that this gene mapped to 3q26 and that it was fused to the acute myeloid leukemia 1 (AML1) gene located at 21q22 in some therapy-related myelodysplastic syndrome patients with 3;21 translocations; however, these fusions actually involve a ribosomal protein L22 pseudogene located at 3q26, and this gene actually maps to 1p36.3-p36.2.

*Estrogen Receptor-$\alpha$ (ESR1)*

The ESR1 gene (HGNG3467, Gene ID: 2099, GenBank: X03635.1, NG_008493.1) encodes an estrogen receptor, a ligand-activated transcription factor composed of several domains important for hormone binding, DNA binding, and activation of transcription. The protein localizes to the nucleus where it may form a homodimer or a heterodimer with estrogen receptor 2. Estrogen and its receptors are essential for sexual development and reproductive function, but also play a role in other tissues such as bone. Estrogen receptors are also involved in pathological processes including breast cancer, endometrial cancer, and osteoporosis. Alternative promoter usage and alternative splicing result in dozens of transcript variants, but the full-length nature of many of these variants has not been determined. The ESR1 gene is located on chromosome 6.

*CSDE1*

The CSDE1 gene (HGNC: 29905, Gene ID: 7812, NC_000001.11) encodes a RNA binding protein called cold shock domain containing E1. This protein is required for internal initiation of translation of human rhinovirus RNA, and it may be involved in translationally coupled mRNA turnover. CSDE1 has also been implicated with other RNA-binding proteins in the cytoplasmic deadenylation/translational and decay interplay of the FOS mRNA mediated by the major coding-region determinant of instability (mCRD) domain.

*SGK1*

The SGK1 gene (HGNC: 10810, Gene ID: 6446, NC_000006.12) encodes a serine/threonine protein kinase that plays an important role in cellular stress response. This kinase activates certain potassium, sodium, and chloride channels, suggesting an involvement in the regulation of processes such as cell survival, neuronal excitability, and renal sodium excretion. High levels of expression of this gene may contribute to conditions such as hypertension and diabetic nephropathy. Several alternatively spliced transcript variants encoding different isoforms have been noted for this gene.

**Next Generation Sequencing of Microsatellite Instability:**

NGS reads from tumors with MSI produce a pattern of dropouts in the alignment process that are characteristic and distinct from other sequence alterations. As disclosed herein, this finding has been utilized to design a method to type MSI status and compared the results to the gold-standard PCR-based capillary electrophoresis sizing method.

The current gold standard method for MSI relies on visual inspection of PCR peaks produced by capillary electrophoresis. In one aspect, the disclosed invention provides a method that defines MSI based on alignment patterns from NGS reads as dedined in the appended claims. Differences in coverage in sequencing reads in normal/non-neoplastic versus tumor samples from tissue samples from patients with cancer can be mathematically calculated based on the NGS data.

Prior to the analysis, the sequencing data is processed to identify the start and end indices of short tandem repeat (STR) nucleotide regions within the sequencing reads. Various tool and programs for identifying STRs are known in the art and may be available online. A programming script can be used to create a file containing all the regions of interest (ROI) from the assay. The script can then identify tandem repeats and returns a list of STR regions and the indices of these regions.

In one embodiment, the method comprises extracting coverage and total read values from the sequence read file only for nucleotides within the indices returned by a tandem repeat finder. In some embodiments, the following algorithm (Eq. 1) is implemented, once for the normal sample (n) and once for the tumor sample (t) beginning at the indexed nucleotide repeat (e.g. the highlighted area of the "Consensus Sequence" row in Figure 4):

$$(\text{Eq. 1}) \quad Dn_i = \left| (C_i - TR_i)/TR_i \right| *100$$

Equation 1 (Eq 1.) can calculate the divergence (D) of the normal sample by subtracting the coverage of nucleotide position (i) in the sequencing reads from the total number of reads (TR) detected in region of interest (ROI), then divide by TR and take the absolute value. Multiplying the result by 100 gives a positive integer value between 1-100. Equation 2 (Eq 2.) can then perform the same calculation on the tumor sample (t).

$$(\text{Eq. 2}) \quad Dt_i = \left| (C_i - TR_i)/TR_i \right| *100$$

$$(\text{Eq. 3}) \quad \Delta D_i = \left| Dt_i - Dn_i \right|$$

$$(\text{Eq. 3}) \quad \sum \Delta D_{i(STR)}$$

Equation 3 (Eq 3.) can calculate the delta divergence (ΔD) for each nucleotide position (i) by subtracting tumor divergence (Dt) from normal divergence (Dn) and taking the absolute value. Equation 4 (Eq 4.) calculates the sum of all ΔD values (Riemann sum). The value obtained represents a quantification of sequence divergence between a normal sample and a tumor sample at the microsatellite and thus represents the level of MSI at that loci.

In some embodiments, the disclosed methods for detecting MSI is performed on known loci of MSI. In some embodiments, the disclosed methods for detecting MSI may be used to identify novel microsatellite loci in any given tumor sample.

In some embodiments, the NGS methods for MSI determination provided herein can accurately quantify microsatellite instability in normal-tumor paired samples, when compared to traditional PCR-based methods.

In some embodiments, the NGS methods for MSI determination provided herein detects novel regions of microsatellite instability in the genome.

In some embodiments, the NGS methods for MSI determination provided herein can simultaneously detect mutations and MSI status in a single assay. In some embodiments, the NGS methods for MSI determination provided herein determines MSI status for purposes of therapy selection in colorectal carcinoma (CRC) or endometrial cancer (EC).

**Methods for Determining Limited Gene Set Subtypes in Cancer:**

Disclosed herein are novel methods for identifying and developing Limited Gene Set subtypes of cancer that may be useful in determining various aspects of patient prognosis including likelihood of recurrence and/or remission and likelihood of progression-free survival, and these methods may be instructive regarding the best course of treatment and/or the timing of treatment for a given patient.

In some embodiments, MSI status and mutation data from various databases may be incorporated. In some embodiments, mutation status or genetic/molecular alterations are converted into quantifiable features. For instance, mutation status for any gene can be considered a binary feature, either mutant or non-mutant, and MSI status can be regarded as either 0=MSS, 1=MSI-low, and 2=MSI-high.

Data mining software can be used to build a Naïve-Bayes model to predict the molecular subgroups from in a given dataset, and feature selection may be performed on the dataset using a chi-square feature selection method or other appropriate statistical methods known in the art. The feature selection method will indicate the models with the best model accuracy, and subgroups may be divided based on a number of features. For instance, data analysis may reveal that a model comprising 5 genes and MSI status (6 features total) has the best model accuracy. A model may comprise between 1-50 features. For instance, a model may incorporate about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 features.

In one exemplary embodiment, the five most informative genes for subtype classification in EC (in order of significance) may be TP53, POLE, PTEN, FBXW7 and RPL22. In some embodiment, these 5 genes and MSI status can be used as features to predict sub-clusters in a Naive-Bayes classification experiment.

Average accuracy for subtype prediction need not be 100% in order to provide clinical benefit. For instance, subtype prediction may be 80, 81, 82, 83, 84, 85, 86, 87, 88, 89, 90, 91, 92, 93, 94, 95, 96, 97, 98, 99, or 100% accurate. In some embodiments, the subtype prediction model is preferably >90% accurate. For instance, in one exemplary embodiment, a 6 feature model for classifying EC was 94% accurate in determining identified subtypes of EC, with average precision and recall of $0.971\pm0.07$ and $0.970\pm0.04$, respectively. The maximum false positive rate was 0.11.

In some embodiments, the NGS methods for MSI determination provided herein can simultaneously detect mutations and MSI status in a single assay. In other embodiments, these methods may be used to identify subgroups of cancer patients with similar prognostic outcomes base on the molecular profile determined via the disclosed methods. In some embodiments, these methods may be used for the purpose of guiding therapy selection in colorectal carcinoma (CRC) or endometrial cancer (EC), including choice of therapy and timing of therapy.

In some embodiments, the methods of combining NGS-derived MSI status and mutation profiles are used to derive novel cancer molecular typing and prognostic models. For example, provided herein is a 5-gene + MSI status model for molecular subtyping of endometrial cancer.

**Endometrial Cancer Staging and Molecular Cluster:**

Endometrial cancer (EC) encompasses the common endometrioid histologic subtype, with variable clinical outcomes, and the less common papillary serous/clear cell carcinoma (PSC), with uniformly adverse prognosis. The primary unmet diagnostic need in EC is to identify cases of low-stage endometrioid histology that have risk of recurrence and would benefit from adjuvant chemotherapy, or therapy at an earlier stage than would previously have been administered prior to the present invention. For instance, some subgroups of subjects with stage I/II endometrial cancer (e.g. those with ESR1 mutations) may benefit from receiving chemotherapy after surgical removal of a tumor even if the subject appears to be in remission.

Four distinct molecular clusters of EC have been identified. These included a group of POLE-mutated cases with an extremely high mutation rate and favorable prognosis and a group of mostly but not exclusively PSC cases with TP53 mutations, frequent genomic copy-number (CN) changes and poor prognosis. However, most cases (66.8%) present with more variable outcomes. These case include groups with endometrioid EC with unmutated TP53 and few CN changes or groups with microsatellite instability (MSI). Disclosed herein are details of how mutation pattern, MSI status, total number of CN alterations, and mutation load interact to predict outcome and recurrence in the clinically relevant stage I/II endometrioid cases.

Staging is the process in which a doctor will classify a tumor based on observational information and how much the cancer may have spread. Factors that are considered in staging include the extent of the tumor, whether the cancer has spread to lymph nodes, and whether it has spread to distant sites. The stage of an endometrial cancer is the most important factor in choosing a treatment plan, and the cancer is generally staged based on examination of tissue removed during an operation. This type of surgical staging may also be paired with other diagnostic techniques, such as ultrasound, MRI, or CT scan to look for signs of spreading. However, this type of staging fails to take into account the molecular profile of a specific individual's cancer.

For instance, while many individuals with stage I/II endometrial cancer may be given a positive prognosis based on staging alone, some of these individuals will ultimately have disease recurrence and some may die from disease progression. Provided herein are methods of creating models for predicting prognosis, recurrence, and/or survival based on the molecular profile of an individual's cancer. In one embodiment, such a method of prognosis may comprise determining whether an individual with stage I/II endometrial cancer has a mutation in the ESR1 gene.

**Predictive Prognostic Molecular Cluster in EC:**

Data analysis may be performed on a set of tumor samples in order to determine whether a prognostic molecular cluster is present. For example, samples from 232 individuals with EC were examined, including 155 cases of an endometrioid subset, and of those cases, 127 cases were stage VII. Prognostic information that may be examined in a given data set may include, but is not limited to, recurrence and outcome. For endometrial cancer, the overall recurrence rate was 19% (45/232), with 23 deaths (10%) reported. In the entire data set, recurrence was more common in the CN-high group (22/60; 37%) and did not occur in the POLE-mutated group.

Once prognostic information has been determined, a model can be developed to identify specific subsets of patients that have similar outcomes based on their molecular profile.

For endometrial cancer, a model filtered for the 5 most significantly mutated (chi-squared) genes and MSI status. This model could predict four previously reported outcome clusters with 96% accuracy. However, Kaplan-Meier analysis showed no significant outcome prediction power for binary MSI status and CN class when analysis was restricted to the stage I/II endometrioid subgroup (p=0.41). This is a subgroup of patients that is of interest because most of them will not receive aggressive chemotherapy to prevent recurrence following surgical intervention; thus, identifying only those individuals that would benefit from chemotherapy would be clinically valuable.

The number of mutations per case was significantly lower in low-stage cases (p<0.01) but did not correlate with recurrence. Commonly mutated oncogenes in EC, including PTEN and PIK3CA, were not significantly differentially mutated by clinical stage or recurrence status in endometrioid cases.

However, several other genes not previously well-studied in EC were differentially mutated in the stage I/II endometrioid subgroup. These included the estrogen receptor-$\alpha$ gene (ESR1), in which mutations were differentially associated with recurrence (p < 0.01, Fisher's exact). Higher CN alteration scores on microarray analysis were also significantly associated with recurrence in that subgroup (p < 0.01, Student's t-test). Genomic complexity in low-stage endometrioid cases was not associated with TP53 mutation (124/127 unmutated) or TP53 loss (126/127 with no deletion), implicating other genome maintenance alterations.

Genomic complexity and mutation status of a small set of genes, including ESR1, CSDE1, and SGK1, are promising recurrence risk predictors in low-stage endometrioid tumors. ESR1 mutation was previously identified to be a marker of aggressive disease in metastatic breast cancer, but was unidentified in EC. Thus, ESR1, CSDE1, and SGK1 can be used to predict prognosis in individuals with stage I/II endometrial cancer, including likelihood or recurrence and progression-free survival.

Accordingly, in some embodiments, the present disclosure provides for methods of prognosing a subject with endometrial cancer comprising: obtaining a sample from the subject; testing the sample for a mutation in the ESR1, CSDE1, and/or SGK1 genes; and indicating the subject will experience progression-free survival if the ESR1, CSDE1, and/or SGK1 genes are not mutated or the subject will have a decreased chance of progression-free survival if the ESR1, CSDE1, and/or SGK1 genes are mutated.

In other embodiments, the present disclosure provide for methods of predicting recurrence in a subject with endometrial cancer comprising: obtaining a sample from the subject; testing the sample for a mutation in the ESR1, CSDE1, and/or SGK1 genes; and indicating the subject will experience cancer recurrence if the ESR1, CSDE1, and/or SGK1 genes are mutated and the subject will not experience cancer recurrence if the ESR1, CSDE1, and/or SGK1 genes are not mutated.

In one embodiment, subjects with stage I/II endometrial cancer that would not traditionally be administered chemotherapy are administered with chemotherapy based on the presence of an ESR1, CSDE1, and/or SGK1 mutation. In another embodiment, subjects that are non-symptomatic may be administered an appropriate therapeutic agent, like chemotherapy, based on the presence of an ESR1, CSDE1, and/or SGK1 mutation. In another embodiment, subjects that are in remission may be administered an appropriate therapeutic agent, like chemotherapy, based on the presence of an ESR1, CSDE1, and/or SGK1 mutation.

In one embodiment, an appropriate therapeutic agent is administered to a subject based on the subject's molecular profile in order to eliminate cancer or reduce the size of a tumor or the number of tumors in a subject; arrest or slow the growth of a tumor in a subject; inhibit or slow the development of a new tumor or tumor metastasis in a subject; and/or decrease the frequency or severity of symptoms and/or recurrences in a subject who currently has or who previously has had cancer. In some embodiments, the subject's molecular profile may include a mutation in the ESR1, CSDE1, and/or SGK1 genes.

In some embodiment, ESR1, CSDE1, and SGK1 may be assayed together to determine mutation status of each gene in a single test. Such a combined test may be useful in determining patient prognosis, risk of recurrence, overall survival, progression free survival, and/or be useful in guiding treatment for the patient. In other embodiments, ESR1, CSDE1, and SGK1 may be assayed separately to determine mutation status of each gene in separate tests.

In one embodiment, an appropriate therapeutic agent is administered to a subject based on the subject's molecular profile in order to minimize the chance that a subject will develop cancer or to delay the development of cancer. For example, a person at increased risk for cancer, as described above, would be a candidate for therapy to prevent cancer. In some embodiments, the subject's molecular profile may include a mutation in ESR1.

EXAMPLES

*Example 1. Cluster Prediction Model*

Data analysis was performed on cases with non-missing recurrence status for the full 232 cases of EC, including a CN-low/MSI subset (155 cases), and a stage I/II subset (127 cases). Prognostic information was provided in a data set including recurrence and outcome. The overall recurrence rate was 20% (45/232), with 23 deaths (10%) reported. Recurrence was significantly more common in the CN-high cluster compared to the CN-low/MSI combined clusters (p < 0.01, Fisher's exact). There were no recurrences in the POLE-mutated group.

Mutation status for any gene was regarded as a binary feature and MSI status was nominal. MSI status and 5 genes were chosen for further examination based on a chi-square attribute selection method with respect to the outcome clusters (classes). The resulting model could predict the four outcome clusters reported in a prior UCEC study (The Cancer Genome Atlas Research Network, *Integrated genomic characterization of endometrial carcinoma,* 00 Nature, 1-8 (2013)) with 94% accuracy (Figure 1).

In a 10-fold cross validation experiment, the model was randomly seeded for 100 iterations and performance metrics were recorded. The average precision and recall were $0.971\pm0.07$ and $0.970\pm0.04$, while the maximum false positive rate was 0.11; these data indicated satisfactory performance.

*Example 2. Stage I/II CN-low/MSI Recurrence Predictors*

When analysis was restricted to the stage I/II CN-low/MSI subgroup there was no outcome prediction power based on mutation status, MSI status, and CN class (Kaplan-Meier, P=0.41).

Higher CN alteration scores on microarray analysis were significantly associated with recurrence in the stage I/II CN-low/MSI subgroup (P<0.01, Student's t-test). Genomic complexity in low-stage CN-low/MSI cases was not associated with TP53 mutation (124/127 unmutated) or TP53 loss (126/127 with no deletion), which implicates other genome maintenance alterations in this subset.

Mutation load (total number of mutations per case), was significantly lower in stage I/II compared to stage III/IV (P<0.01); however, the number of mutations per case was not significantly correlated with recurrence in the low-stage CN-low/MSI cohort. Mutation rate in previously characterized oncogenes commonly mutated in EC, including PTEN, FGFR2, and PIK3CA, did not differ based on clinical stage or recurrence status in stage I/II CN-low/MSI cases (P=0.70, 0.22, 0.29, respectively).

Several genes that were identified that were not previously known in EC and that were mutated at a significantly higher rate in the group of cases that recurred within the stage I/II CN-low/MSI subgroup (Figure 2, bottom-left). One of these genes was the estrogen receptor-$\alpha$ gene (ESR1) (p < 0.01, Fisher's exact). Of the three ESR1 mutations in the recurrence group, two were Y537 substitutions and one was an in-frame deletion, GKC415del; both mutations were located in the ligand-binding domain (LBD). Y537 substitutions have been previously reported as activating mutations in breast cancer and were not found in the 390 ER-positive breast cancers from the TCGA study. ESR1 mutations are a modest indicator of progression-free survival when looking at the full UCEC data set (Figure 3A). However, there is a significant difference between wild-type and mutant ESR1 in the Stage I/II CN-low/MSI subset (Kaplan-Meier, P<0.01) (Figure 3B).

*Example 3. Method for Accurate MSI Typing and Mutation Profiling in a Single Sequencing Assay for Prognostic and Theranostic Modeling in Cancer Samples*

MSI was determined based on alignment patterns from NGS reads. Differences in coverage in sequencing reads in normal/non-neoplastic versus tumor samples from tissue samples from patients with cancer are mathematically calculated.

Prior to the analysis, the sequencing data is processed using a tool "Tandem Repeats Finder" (TRF) (http://tandem.bu.edu/trf/trf.html) to identify the start and end indices of short tandem repeat (STR) nucleotide regions within the sequencing reads. A programming script created a .fasta file containing all the regions of interest (ROI) from the assay. The script then executed TRF and returns a list of STR regions and the indices of these regions.

Coverage and total read values were extracted from the sequence read file only for nucleotides within the indices returned by TRF.

Beginning at the indexed nucleotide repeat (highlighted in the "Consensus Sequence" row of Figure 4), divergence algorithms were implemented, once for the normal sample (n) and once for the tumor sample (t). (Figure 4)

Equation 1 (Eq 1.) calculated the divergence (D) of the normal sample by subtracting the coverage of nucleotide position (i) in the sequencing reads from the total number of reads (TR) detected in region of interest (ROI), and then divided by TR and take the absolute value. Multiplying the result by 100 gave a positive integer value between 1-100. Equation 2 (Eq 2.) was then used to perform the same calculation on the tumor sample (t).

Equation 3 (Eq 3.) calculated the delta divergence ($\Delta$D) for each nucleotide position (i) by subtracting tumor divergence (Dt) from normal divergence (Dn) and then taking the absolute value. Equation 4 (Eq 4.) calculated the sum of all $\Delta$D values (Riemann sum). This value obtained represented a quantification of sequence divergence between normal and tumor at the microsatellite and thus represents the level of MSI at that loci.

A Riemann sum correlation was determined in order to validate the method by performing sequencing on tumor cell lines with known MSI status. The colon cancer cell HCT-116, known to a have high level of MSI, was classified by this method as MSI in the ROI microsatellites. In contrast, the microsatellite stable colon cancer cell line SW480 demonstrated no MSI by this method. Table 1 shows the validation results for the microsatellite BAT-25 and Table 2 shows the cell line validation results for the microsatellite BAT-26. Parallel analysis of these cell lines by the PCR-CE method confirmed the known status of the tested lines (not shown).

**Table 1: Reimann sum for the BAT-25 microsatellite in dilution studies with HCT-116 (MSI-high) and SW480 (MSS) cell lines.**

| BAT-25 | | | |
|---|---|---|---|
| Riemann sum | HCT-116 up | SW480 ul | Dilution Ratio |
| 445.6 | 15 | 0 | 100%-0% |
| 402.5 | 14.85 | 0.15 | 99%-1% |
| 357.6 | 14.25 | 0.75 | 95%-5% |
| 377.65 | 13.5 | 1.5 | 90%-10% |
| 365.3 | 11.25 | 3.75 | 75%-25% |
| 286.5 | 7.5 | 7.5 | 50%-50% |
| 179.1 | 3.75 | 11.25 | 25%-75% |
| 40.1 | 1.5 | 13.5 | 10%-90% |
| 82.8 | 0.75 | 14.25 | 5%-95% |
| 40 | 0.15 | 14.85 | 1%-99% |

**Table 2: Reimann sum for the BAT-26 microsatellite in dilution studies with HCT-116 (MSI-high) and SW480 (MSS) cell lines.**

| BAT26 | | | |
|---|---|---|---|
| Riemann sum | HCT-116 ul | SW480 ul | Dilution Ratio |
| 755 | 15 | 0 | |
| 758 | 14.85 | 0.15 | 99%-1% |
| 725.65 | 14.25 | 0.75 | 95%-5% |
| 772.2 | 13.5 | 1.5 | 90%-10% |
| 802.1 | 11.25 | 3.75 | 75%-25% |
| 780.7 | 7.5 | 7.5 | 50%-50% |
| 671.6 | 3.75 | 11.25 | 25%-75% |
| 658.3 | 1.5 | 13.5 | 10%-90% |
| 721.5 | 0.75 | 14.25 | 5%-95% |
| 452.74 | 0.15 | 14.85 | 1%-99% |

The BAT-25 study demonstrated near equal coverage for each sample in the dilution study. Higher Reimann sum scores were well correlated with higher concentrations of HCT-116 DNA for BAT-25 ($r^2 = 0.95$). BAT-26, however, showed more variable coverage for some dilution samples (ROI dropout), which affected the Reimann sum correlation for BAT-26 ($r2 = 0.44$).

Since, the expansion/contraction pattern is distinct for each microsatellite, a Reimann sum threshold for calling MSI for each ROI was needed, and was obtained by averaging a number of samples with known MSI status.

The Reimann sum score method was performed on all microsatellite ROIs included in the sequencing run. The results for all microsatellite/ROIs were combined to give the final MSI call for each case, using the same criteria as the PCR-CE method.

The validation results indicate that this method can also be used to identify novel microsatellite loci in any given tumor sample.

Figure 5 shows a histogram image from JSI-SeqNext software (A) showing sequencing coverage of a normal sample, the black arrows indicate two microsatellite areas. Also shown is an image produced from the MSI detection method (B). The nucleotide position (i) of the amplicon/region of interest is located on the x axis. Black arrows serve as a reference for accurate representation of the coverage histogram. Normal sample coverage (upper line) and tumor sample coverage (middle line) are measured in number of nucleotides (y1 axis). Delta divergence (lower line) (Eq. 3) is indicated by the integer values on the y2 axis.

Figure 6 shows an example of a sample showing a microsatellite stable region of interest (ROI). The tumor (T) and normal (N) samples were run by capillary electrophoresis for five MSI markers (top). A unimodal peak is visible at high power (bottom left) in both the tumor and normal samples, indicating a stable microsatellite (MSS) pattern. The MSI detection method (bottom right) detects a low divergence (lower line), confirming the results.

Figure 7 shows an example of a sample showing a microsatellite instable region of interest (ROI). The tumor (T) and normal (N) samples were run by capillary electrophoresis for five MSI markers (top). A slight bimodal peak is visible at high power (bottom left) in both the tumor and normal samples, indicating a MSI in this region. The MSI detection method (bottom right) detects a high divergence (lower line), confirming the results.

*Example 4. Technical Validation of the Method for MSI Status Determination and Mutation Detection Using NGS in Endometrial Cancer*

To demonstrate of the utility of determining MSI status and mutation status in the NGS sequencing run, an Illumina sequencing panel was designed for endometrial cancer (EC).

EC encompasses the common endometrioid histologic subtype, with variable clinical outcomes, and the less common papillary serous/clear cell carcinoma (PSC), with uniformly adverse prognosis. Microsatellite instability (MSI) is seen in a subset of endometrioid EC and has been recommended as a diagnostic test to detect EC that arise from the hereditary Lynch syndrome. A number of molecular analyses of EC have identified mutation patterns that correlate with histology type and high-risk clinical features that were incorporated into the custom-designed NGS assay.

In particular, the multicenter Uterine Corpus Endometrial Carcinoma (UCEC) study identified 4 distinct molecular clusters of EC. These clusters included 1) a group of *POLE*-mutated cases associated with an extremely high mutation rate and favorable prognosis and 2) a group of mostly, but not exclusively, PSC cases associated with *TP53* mutations, frequent genomic copy-number (CN) changes, and poor prognosis. However, most of the cases (155/232, 66.8%) were 3) endometrioid EC with unmutated *TP53* and few CN changes, or 4) cases exhibiting microsatellite instability (MSI). These last 2 groups had more variable outcomes.

A panel was designed to simultaneously determine the MSI status and detect mutations that can classify EC into known molecular sub-groups, including POLE-mutated, CN-low, CN-high variants with mutations in the PIK3/RAS pathway and MSI cases.

In order to determine these subgroups, the following studies were performed:

(1) a custom NGS panel was designed for use with the Illumina TruSeq method that contained 19 commonly mutated genes in solid tumors as well as amplicons spanning the 5 NCI microsatellite loci. Genes were chosen for mutation analysis based on their frequency and class association with EC;

(2) DNA sequencing was performed using the mutation/microsatellite custom panel on paired normal and tumor samples from primary colorectal and endometrial cancer samples as well as known MSI+ and MSI- CRC cell lines;

(3) the approach was bioinformatically validated for detecting MSI in NGS data using simulated sequence reads; and

(4) MSI status determination was compared between the disclosed NGS method and the traditional capillary gel electrophoresis method.

Table 3 lists the genes, regions covered, and amount of base pairs sequenced in a custom Illumina DNA-based NGS assay for endometrial cancer.

**Table 3: NGS Assay Genes and Regions**

| Gene chosen for panel | Target area | Base pair |
|---|---|---|
| **Characteristic of high-grade EC** | | |
| **TP53** | CDS | 1182 |
| **PPP2R1A** | hotspot | 400 |
| **RAS pathway genes** | | |
| **PTEN** | CDS | 1212 |
| **KRAS** | CDS | 500 |
| **NRAS** | CDS | 500 |
| **PIK3CA** | hotspot | 2000 |
| **PIK3R1** | hotspot | 1000 |
| **Genes chosen based on mutation frequency in EC or prognostic associations** | | |
| **CTNNB1** | hotspot | 250 |
| **CTCF** | CDS | 2184 |
| **FBXW7** | CDS | 2124 |
| **SPOP** | CDS | 1125 |
| **SOX17** | CDS | 1245 |
| **FGFR2** | CDS | 2466 |
| **RPL22** | hotspot | 250 |
| **ARID1A** | CDS | 6858 |
| **POLE** | hotspot | 750 |
| CCAT2 | 1 amplicon | 200 |
| **Microsatellite loci** | | |
| BAT-26 | 1 amplicon | 100 |
| BAT-25 | 1 amplicon | 150 |

(continued)

| Microsatellite loci | | |
|---|---|---|
| D2S123 | 1 amplicon | 250 |
| D5S346 | 1 amplicon | 150 |
| D17S250 | 1 amplicon | 150 |

*Example 5. Using the Combined MSI Typing-Mutation Assay Method to Build a Limited Gene Set Model for Endometrial Cancer Typing.*

In addition to designing and validating the MSI/mutation NGS EC assay, the ability of the disclosed methods to find the 4 molecular subtypes of EC defined by The Cancer Genome Atlas Research Network was assessed.

To accomplish this, the data from UCEC study was utilized as a training set. In that study, mutation data was obtained by NGS and the MSI status was determined by the standard PCR-CE method. Once the minimal gene set needed to encode accurate subtyping was determined from UCEC data, this model was tested against primary EC tumor sequencing data obtained using the custom-designed Illumina assay.

Methods:

MSI status and mutation data from the genes in the custom panel from the UCEC dataset were downloaded from cbioportal.org. (Table 3). Mutation status for any gene was considered a binary feature, either mutant or non-mutant. MSI status was regarded as either 0=MSS, 1=MSI-low, and 2=MSI-high. These results were compared to the UCEC-derived molecular EC subgroup, POLE, CN-low, CN-high, and MSI.

Weka, a data mining software, was used to build a Naïve-Bayes model to predict the molecular subgroups from the UCEC dataset. Feature selection was performed on the dataset using a chi-square feature selection method. The feature selection method with the best model accuracy was a 5 gene and MSI status model (6 features total). The five most informative genes for subtype classification in the UCEC data were (in order of significance) *TP53, POLE, PTEN, FBXW7* and *RPL22.*

These 5 genes and MSI status were used as features to predict sub-clusters in a Naive-Bayes classification experiment. The order of cases was randomized and a Naïve-Bayes classifier was applied to the dataset in 100 randomly seeded 10-fold cross validation experiments.

Average accuracy for subtype prediction was 94%; average precision and recall were $0.971 \pm 0.07$ and $0.970 \pm 0.04$, respectively. The maximum false positive rate was 0.11.

This model was then tested against the sequencing data from 30 primary EC cases obtained from the custom panel.

*Example 6. Mutations in ESR1, CSDE1 and SGK1 as Predictors of Poor Outcome in Low-Stage Endometrial Cancer*

Endometrial cancer (EC) encompasses the common endometrioid histologic subtype, with variable clinical outcomes, and the less common papillary serous/clear cell carcinoma (PSC), with uniformly adverse prognosis. A primary unmet diagnostic need in EC is the identification of cases with endometrioid histology and low-stage that have risk of recurrence and would benefit from adjuvant chemotherapy or radiotherapy. Microsatellite instability (MSI) is seen in a subset of endometrioid EC and has been recommended as a diagnostic test to detect hereditary Lynch syndrome. A number of molecular analyses of EC have identified mutation patterns that correlate with histology type and high-risk clinical features but no well-accepted predictive biomarkers for the endometrioid subtype have yet emerged.

The multicenter Uterine Corpus Endometrial Carcinoma (UCEC) study recently employed expression and genomic microarrays, methylation profiling, and next-generation sequencing (NGS). Based primarily on the sequencing and microarray data, the study identified 4 distinct molecular clusters of EC. These clusters included 1) a group of *POLE*-mutated cases associated with an extremely high mutation rate and favorable prognosis and 2) a group of mostly, but not exclusively, PSC cases associated with *TP53* mutations, frequent genomic copy-number (CN) changes, and poor prognosis. However, most of the cases (155/232, 66.8%) were 3) endometrioid EC with unmutated *TP53* and few CN changes, or 4) cases exhibiting microsatellite instability (MSI). These last 2 groups had more variable outcomes limiting their utility in routine outcome prediction.

That initial analysis of the UCEC data did not clearly identify a genetic, expression or epigenetic signature for stratifying Stage I/II endometrioid EC. The UCEC data was analyzed and three mutated genes were identified that can stratify outcome in low-stage EC, in univariate analysis. One of these genes *ESR1*/estrogen receptor is potentially targetable with a range of currently available therapeutics.

Methods:

*Analysis of outcome in the UCEC data set*

Regardless of molecular subgroup, advanced stage (III/IV) endometrial cancer in UCEC data showed poor outcome. The analysis was thus limited to Stage I/II cases. Of these cases, the *POLE*-mutated subgroup was shown to have a highly favorable outcome and the CN-high subgroup are characterized to have an inferior outcome. Those two subgroups comprised only 95/232 (41%) of the total cases. The study thus further focused just on the Stage I/II, CN-low/MSI subgroup, which contains a majority of the UCEC cases and had the more variable outcomes.

Outcome data included overall survival (OS) and recurrence, which was encoded as a binary feature as recurred or progression-free. Tables 1-3 show the categories used for analysis.

*Statistical Analysis*

For the reasons above, only Stage VII, CN-low/MSI cases with recurrence data (127) were selected for further analysis.

Mutations from the 82 most recurrently mutated genes and clinical data for all 232 UCEC cases were organized table format. Mutations were translated to a binary value, mutated or non-mutated.

In this group, most of the cases (111) were progression-free, with 16 recurrences. The distribution of CN-low and MSI EC subtypes was similar between the recurrence and progression-free groups. A Fisher's exact test (2x2 contingency table) was performed for each gene.

Results:

OS was not significantly different for any gene in the Stage I/II, CN-low/MSI cases, likely due to the few deaths reported.

For progression, there were three genes that were significantly differentially mutated in the patients with recurrence as compared to the progression-free group. These were *ESR1, CSDE1* and *SGK1.*

Figure 8 shows results of the Fisher's exact test for each of the three genes where mutation was significantly correlate with recurrence in the Stage I/II, CN-low/MSI subgroup

While the invention has been described and exemplified in sufficient detail for those skilled in this art to make and use it, various alternatives, modifications, and improvements should be apparent without departing from the spirit and scope of the invention.

One skilled in the art readily appreciates that the present invention is well adapted to carry out the objects and obtain the ends and advantages mentioned, as well as those inherent therein. Modifications therein and other uses will occur to those skilled in the art.

It will be readily apparent to a person skilled in the art that varying substitutions and modifications may be made to the invention disclosed herein without departing from the scope of the invention as defined in the appended claims.

All patents and publications mentioned in the specification are indicative of the levels of those of ordinary skill in the art to which the invention pertains.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Non-limiting embodiments are set forth within the following claims.

References:

Arabi H et al._Impact of microsatellite instability (MSI) on survival in high grade endometrial carcinoma. Gynecol Oncol 2009 May;113(2):153-8.

Benson G, et al, Tandem repeats finder: a program to analyze DNA sequences. Nuc Acids Res1999; 27(2):573-580.

Cirisano,F.D, et al. (2000). The outcome of stage I-II clinically and surgically staged papillary serous and clear cell endometrial cancers when compared with endometrioid carcinoma. Gynecologic oncology, 77(1), 55-65.

Creutzberg CL, et al. Nomograms for Prediction of Outcome With or Without Adjuvant Radiation Therapy for Patients With Endometrial Cancer: A Pooled Analysis of PORTEC-1 and PORTEC-2 Trials. International Journal of Radiation Oncology* Biology* Physics 91.3 (2015): 530-539.

Diaz-Padilla I, et al. Mismatch repair status and clinical outcome in endometrial cancer: a systematic review and meta-analysis. Crit Rev Oncol Hematol 2013 Oct;88(1):154-67.

Deschoolmeester V, et al. Comparison of three commonly used PCR-based techniques to analyze MSI status in sporadic colorectal cancer J Clin Lab Anal 2006;20(2), 52-61.

Giardiello EIM, et al. Guidelines on genetic evaluation and management of Lynch syndrome: a consensus statement by the US Multi-society Task Force on colorectal cancer. Am J Gastroenterol_2014 Aug;109(8):1159-79.

Gould-Suarez M, et al._Cost-effectiveness and diagnostic effectiveness analyses of multiple algorithms for the diagnosis of Lynch syndrome. Dig Dis Sci_2014 Dec;59(12):2913-26.

Hogberg T. Adjuvant Chemotherapy in Endometrial Carcinoma: Overview of Randomised Trials. Clinical Oncol. 2008;20(6): 463-469.

Kim, TM, Laird, PW, & Park, PJ. The landscape of microsatellite instability in colorectal and endometrial cancer genomes. Cell 2013;155(4):858-868.

Mills AM, et al. Lynch syndrome screening should be considered for all patients with newly diagnosed endometrial cancer. Am J Surg Pathol 2014 Nov;38(11):1501-9.

Missiaglia E, et al. Distal and proximal colon cancers differ in terms of molecular, pathological, and clinical features. Ann Oncol. 2014 Oct;25(10):1995-2001

Modica I, et al. Utility of immunohistochemistry in predicting microsatellite instability in endometrial carcinoma. Am J Surg Pathol 2007 May;31(5):744-51.

Nardon E, et al. A Multicenter Study to Validate the Reproducibility of MSI Testing With a Panel of 5 Quasimonomorphic Mononucleotide Repeats. Diag Molec Pathol. 2010;19(4): 236-242.

Niu, B, et al. MSIsensor: microsatellite instability detection using paired tumor-normal sequence data. Bioinformatics 2014;30(7):1015-1016.

Popat S, Hubner R and Houlston RS. Systematic Review of Microsatellite Instability and Colorectal Cancer Prognosis. J Clin Oncol 2005 Jan 20; 23(3) 609-618.

The Cancer Genome Atlas Research Network. Integrated genomic characterization of endometrial carcinoma. Nature 2013 May 02;497:67-73. May 02, 2013.

**Claims**

1. A method of determining microsatellite instability (MSI) of a DNA sample comprising,

   a) obtaining a tumor sample and a normal sample from a subject, wherein each sample comprises DNA;
   b) sequencing a microsatellite location in the DNA of each sample using next generation sequencing (NGS);

c) identifying tandem repeats in the sequences;
d) extracting coverage and total read values from the sequences comprising tandem repeats;
e) calculating the divergence of the normal sample and the tumor sample;
f) calculating the delta divergence for each nucleotide position;
g) calculating the sum of all delta divergence values; wherein the value obtained from the sum of all delta divergence values represents the quantification of sequence divergence between the normal sample and the tumor sample at the sequenced microsatellite location.

2. The method of claim 1, wherein more than one microsatellite location is sequenced at a time.

3. The method of and of claims 1 or 2 further comprising testing the sample for a mutation in the ESR1 gene.

4. The method of claim 3, wherein the mutation in the ESR1 gene is a Y537 substitution.

5. The method of claim 3, wherein the mutation in the ESR1 gene is an in-frame deletion.

6. The method of claim 3, wherein the mutation in the ESR1 gene results in an amino acid substitution in the ESR1 ligand binding domain (LBD), optionally, wherein the mutation in the ESR1 gene results in an in-frame deletion in the ESR1 LBD.

7. The method of claim 3, wherein the mutation in the ESR1 gene is GKC415del.

8. The method of any one of claims 1-7 further comprising testing the sample for a mutation in the CDSE1 gene and the SGK1 gene.

9. The method of any one of claims 1-8, wherein the subject has a tumor or cancer.

10. The method of claim 9, wherein the cancer is endometrial cancer.

11. The method of claim 10, wherein the endometrial cancer is stage I/II endometrial cancer.

12. The method of any one of claims 10 or 11, wherein the subject has previously undergone surgery to remove the tumor or cancer.

13. The method of any one of claims 10-12, wherein the subject is non-symptomatic or in remission.


**Patentansprüche**

1. Verfahren zur Bestimmung der Mikrosatelliteninstabilität (MSI) einer DNA-Probe, umfassend,

a) Beziehen einer Tumorprobe und einer normalen Probe von einem Subjekt, wobei jede Probe DNA umfasst;
b) Sequenzieren einer Mikrosatellitenstelle in der DNA jeder Probe unter Verwendung von Next Generation Sequenzierung (NGS);
c) Identifizieren von Tandemwiederholungen in den Sequenzen;
d) Extrahieren von Deckungs- und Gesamtlesewerten aus den Sequenzen, die Tandemwiederholungen enthalten;
e) Berechnen der Divergenz zwischen der normalen Probe und der Tumorprobe;
f) Berechnen der Delta Divergenz für jede Nukleotidposition;
g) Berechnen der Summe aller Delta Divergenzwerte; wobei der aus der Summe aller Delta Divergenzwerte erhaltene Wert die Quantifizierung der Sequenzdivergenz zwischen der normalen Probe und der Tumorprobe an der sequenzierten Mikrosatellitenposition repräsentiert.

2. Verfahren gemäß Anspruch 1, wobei mehr als eine Mikrosatellitenstelle gleichzeitig sequenziert wird.

3. Verfahren gemäß irgendeinem der Ansprüche 1 oder 2, ferner umfassend testen der Probe auf eine Mutation in dem ESR1 Gen.

**4.** Verfahren gemäß Anspruch 3, wobei die Mutation in dem ESR1 Gen eine Y537 Substitution ist.

**5.** Verfahren gemäß Anspruch 3, wobei die Mutation in dem ESR1 Gen eine In-Frame Deletion ist.

**6.** Verfahren gemäß Anspruch 3, wobei die Mutation in dem ESR1 Gen in einer Aminosäure Substitution in der ESR1 Ligandenbindungsdomäne (LBD) resultiert, optional wobei die Mutation in dem ESR1 Gen in einer In-Frame Deletion in der ESR1 LBD resultiert.

**7.** Verfahren gemäß Anspruch 3, wobei die Mutation in dem ESR1 Gen GKC415del ist.

**8.** Verfahren gemäß irgendeinem der Ansprüche 1-7, ferner umfassend testen der Probe auf eine Mutation in dem CDSE1 Gen und dem SGK1 Gen.

**9.** Verfahren gemäß irgendeinem der Ansprüche 1-8, wobei das Subjekt einen Tumor oder Krebs hat.

**10.** Verfahren gemäß Anspruch 9, wobei der Krebs Endometriumkrebs ist.

**11.** Verfahren gemäß Anspruch 10, wobei der Endometriumkrebs Endometriumkrebs im Stadium I/II ist.

**12.** Verfahren gemäß irgendeinem der Ansprüche 10 oder 11, wobei sich das Subjekt zuvor einer Operation zur Entfernung des Tumors oder Krebses unterzogen wurde.

**13.** Verfahren gemäß irgendeinem der Ansprüche 10-12, wobei das Subjekt nicht symptomatisch ist oder in Remission ist.


**Revendications**

**1.** Procédé de détermination d'une instabilité de microsatellite (MSI) d'un échantillon d'ADN comprenant,

a) l'obtention d'un échantillon de tumeur et d'un échantillon normal à partir d'un sujet, dans lequel chaque échantillon comprend de l'ADN ;
b) le séquençage d'un emplacement de microsatellite dans l'ADN de chaque échantillon en utilisant un séquençage de nouvelle génération (NGS) ;
c) l'identification de répétitions en tandem dans les séquences ;
d) l'extraction des valeurs de lecture de couverture et totale à partir des séquences comprenant des répétitions en tandem ;
e) le calcul de la divergence de l'échantillon normal et de l'échantillon de tumeur ;
f) le calcul de la divergence delta pour chaque position de nucléotide ;
g) le calcul de la somme de toutes les valeurs de divergence delta ; dans lequel la valeur obtenue à partir de la somme de toutes les valeurs de divergence delta représente la quantification de la divergence de séquence entre l'échantillon normal et l'échantillon de tumeur à l'emplacement du microsatellite séquencé.

**2.** Procédé selon la revendication 1, dans lequel plus d'un emplacement de microsatellite est séquencé à la fois.

**3.** Procédé selon la revendication 1 ou la revendication 2 comprenant en outre le test de l'échantillon pour une mutation du gène ESR1.

**4.** Procédé selon la revendication 3, dans lequel la mutation dans le gène ESR1 est une substitution Y537.

**5.** Procédé selon la revendication 3, dans lequel la mutation dans le gène ESR1 est une délétion dans le cadre.

**6.** Procédé selon la revendication 3, dans lequel la mutation dans le gène ESR1 entraîne une substitution d'acide aminé dans le domaine de liaison du ligand (LBD) de ESR1, facultativement, dans lequel la mutation dans le gène ESR1 entraîne une délétion dans le cadre dans le LBD de ESR1.

**7.** Procédé selon la revendication 3, dans lequel la mutation dans le gène ESR1 est GKC415del.

8. Procédé selon l'une quelconque des revendications 1 à 7 comprenant en outre le test de l'échantillon pour une mutation dans le gène CDSE1 et le gène SGK1.

9. Procédé selon l'une quelconque des revendications 1 à 8, dans lequel le sujet présente une tumeur ou un cancer.

10. Procédé selon la revendication 9, dans lequel le cancer est un cancer de l'endomètre.

11. Procédé selon la revendication 10, dans lequel le cancer de l'endomètre est un cancer de l'endomètre de stade I/II.

12. Procédé selon l'une quelconque des revendications 10 ou 11, dans lequel le sujet a préalablement subi une intervention chirurgicale pour retirer la tumeur ou le cancer.

13. Procédé selon l'une quelconque des revendications 10 à 12, dans lequel le sujet est asymptomatique ou en rémission.

UCEC multiplatform study provides novel genomic classification of endometrial tumors.

Model predicts UCEC genomic classification of endometrial tumors using only NGS.

FIGURE 1

FIGURE 2

A

B

**FIGURE 3**

| Consensus Sequence | T | A | C | C | C | C | C | C | G | T | ... | T |
|---|---|---|---|---|---|---|---|---|---|---|---|---|
| | | | Short Tandem Repeat (STR) by *Tandem Repeat Finder* | | | | | | | | | |
| **Matched Normal (n)** | | | | | | | | | | | | |
| nucleotide position in read | $i_1$ | $i_2$ | $i_3$ | $i_4$ | $i_5$ | $i_6$ | $i_7$ | $i_8$ | $i_9$ | $i_{10}$ | ... | $i_{150}$ |
| Coverage (C) | 150 | 150 | 149 | 148 | 146 | 145 | 145 | 145 | 150 | 150 | ... | 150 |
| Total Reads (TR) | 150 | 150 | 150 | 150 | 150 | 150 | 150 | 150 | 150 | 150 | ... | 150 |
| *Normal Divergence (Dn)* | 0.0 | 0.0 | 0.7 | 1.3 | 2.7 | 3.3 | 3.3 | 3.3 | 0.0 | 0.0 | ... | 0 |

Eq 1. $Dn_i = | (C_i - TR_i) / TR_i | * 100$

| **Tumor Sample (t)** | | | | | | | | | | | | |
|---|---|---|---|---|---|---|---|---|---|---|---|---|
| nucleotide position in read | $i_1$ | $i_2$ | $i_3$ | $i_4$ | $i_5$ | $i_6$ | $i_7$ | $i_8$ | $i_9$ | $i_{10}$ | ... | $i_{150}$ |
| Coverage (C) | 200 | 200 | 193 | 180 | 170 | 150 | 145 | 150 | 200 | 200 | ... | 200 |
| Total Reads (TR) | 200 | 200 | 200 | 200 | 200 | 200 | 200 | 200 | 200 | 200 | ... | 200 |
| *Tumor Divergence (Dt)* | 0.0 | 0.0 | 3.5 | 10.0 | 15.0 | 25.0 | 27.5 | 25.0 | 0.0 | 0.0 | ... | 0 |

Eq 2. $Dt_i = | (C_i - TR_i) / TR_i | * 100$

| *Delta Divergence (ΔD)* | 0 | 0 | 2.8333 | 8.6667 | 12.333 | 21.667 | 24.167 | 21.667 | 0 | 0 | ... | 0 |
|---|---|---|---|---|---|---|---|---|---|---|---|---|

Eq 3. $\Delta D_i = | Dt_i - Dn_i |$

*Reimann Sum of STR in ROI*     91.3

Eq 4. $\Sigma \Delta D_{i(STR)}$

(STR) = short tandem repeat region

# FIGURE 4

FIGURE 5

**FIGURE 6**

FIGURE 7

*CSDE1*

|  | mutant | wild-type |
|---|---|---|
| Recurred | 3 | 13 |
| Progression-free | 4 | 107 |
|  |  | *$p < 0.05$ |

*ESR1*

|  | mutant | wild-type |
|---|---|---|
| Recurred | 3 | 13 |
| Progression-free | 1 | 110 |
|  |  | *$p < 0.01$ |

*SGK1*

|  | mutant | wild-type |
|---|---|---|
| Recurred | 3 | 13 |
| Progression-free | 2 | 109 |
|  |  | *$p < 0.05$ |

**FIGURE 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- The Cancer Genome Atlas Research Network, Integrated genomic characterization of endometrial carcinoma. *00 NATURE,* 2013, 1-8
- **SALIPANTE ; STEPHEN J et al.** Microsatellite instability detection by next generation sequencing. *Clinical chemistry,* 2014, vol. 60 (9), 1192-1199
- **ARABI H et al.** Impact of microsatellite instability (MSI) on survival in high grade endometrial carcinoma. *Gynecol Oncol,* May 2009, vol. 113 (2), 153-8
- **BENSON G et al.** Tandem repeats finder: a program to analyze DNA sequences. *Nuc Acids Res,* 1999, vol. 27 (2), 573-580
- **CIRISANO,F.D et al.** The outcome of stage I-II clinically and surgically staged papillary serous and clear cell endometrial cancers when compared with endometrioid carcinoma. *Gynecologic oncology,* 2000, vol. 77 (1), 55-65
- **CREUTZBERG CL et al.** Nomograms for Prediction of Outcome With or Without Adjuvant Radiation Therapy for Patients With Endometrial Cancer: A Pooled Analysis of PORTEC-1 and PORTEC-2 Trials. *International Journal of Radiation Oncology\* Biology\* Physics,* 2015, vol. 91 (3), 530-539
- **DIAZ-PADILLA I et al.** Mismatch repair status and clinical outcome in endometrial cancer: a systematic review and meta-analysis. *Crit Rev Oncol Hematol,* October 2013, vol. 88 (1), 154-67
- **DESCHOOLMEESTER V et al.** Comparison of three commonly used PCR-based techniques to analyze MSI status in sporadic colorectal cancer. *J Clin Lab Anal,* 2006, vol. 20 (2), 52-61
- **GIARDIELLO ELM et al.** Guidelines on genetic evaluation and management of Lynch syndrome: a consensus statement by the US Multi-society Task Force on colorectal cancer. *Am J Gastroenterol,* August 2014, vol. 109 (8), 1159-79

- **GOULD-SUAREZ M et al.** Cost-effectiveness and diagnostic effectiveness analyses of multiple algorithms for the diagnosis of Lynch syndrome. *Dig Dis Sci,* December 2014, vol. 59 (12), 2913-26
- **HOGBERG T.** Adjuvant Chemotherapy in Endometrial Carcinoma: Overview of Randomised Trials. *Clinical Oncol.,* 2008, vol. 20 (6), 463-469
- **KIM, TM ; LAIRD, PW ; PARK, PJ.** The landscape of microsatellite instability in colorectal and endometrial cancer genomes. *Cell,* 2013, vol. 155 (4), 858-868
- **MILLS AM et al.** Lynch syndrome screening should be considered for all patients with newly diagnosed endometrial cancer. *Am J Surg Pathol,* November 2014, vol. 38 (11), 1501-9
- **MISSIAGLIA E et al.** Distal and proximal colon cancers differ in terms of molecular, pathological, and clinical features. *Ann Oncol.,* October 2014, vol. 25 (10), 1995-2001
- **MODICA I et al.** Utility of immunohistochemistry in predicting microsatellite instability in endometrial carcinoma. *Am J Surg Pathol,* May 2007, vol. 31 (5), 744-51
- **NARDON E et al.** A Multicenter Study to Validate the Reproducibility of MSI Testing With a Panel of 5 Quasimonomorphic Mononucleotide Repeats. *Diag Molec Pathol.,* 2010, vol. 19 (4), 236-242
- **NIU, B et al.** MSIsensor: microsatellite instability detection using paired tumor-normal sequence data. *Bioinformatics,* 2014, vol. 30 (7), 1015-1016
- **POPAT S ; HUBNER R ; HOULSTON RS.** Systematic Review of Microsatellite Instability and Colorectal Cancer Prognosis. *J Clin Oncol,* 20 January 2005, vol. 23 (3), 609-618
- The Cancer Genome Atlas Research Network. Integrated genomic characterization of endometrial carcinoma. *Nature,* 02 May 2013, vol. 497, 67-73